# EUROPÄISCHE PATENTSCHRIFT

(11) **EP 2 531 128 B1**
(45) Veröffentlichungstag und Bekanntmachung des Hinweises auf die Patenterteilung: **03.07.2019**
(21) Anmeldenummer: 10794973.7
(22) Anmeldetag: 15.12.2010
(51) Int. Cl.: A61B 18/04

(54) **ELEKTROCHIRURGISCHE ANORDNUNG UND ELEKTROCHIRURGISCHES INSTRUMENT**
ELECTROSURGICAL ASSEMBLY AND ELECTROSURGICAL INSTRUMENT
SYSTÈME ÉLECTROCHIRURGICAL ET INSTRUMENT ÉLECTROCHIRURGICAL

(30) Priorität: 10.03.2010 DE 102010015899; 04.02.2010 DE 102010000305
(43) Veröffentlichungstag der Anmeldung: 12.12.2012
(60) Teilanmeldung: 19167963.8
(73) Patentinhaber: Erbe Elektromedizin GmbH, 72072 Tübingen (DE)
(72) Erfinder: FISCHER, Klaus, 72202 Nagold (DE); NEUGEBAUER, Alexander, 72116 Mössingen (DE); VOIGTLÄNDER, Matthias, 72810 Gomaringen (DE); SCHÄLLER, Daniel, 72070 Tübingen (DE); SZYRACH, Mara, 8052 Zürich (DE); KRONENTHALER, Jörg, 72145 Hirrlingen (DE); BLOBEL, Lars, 72119 Ammerbuch-Entringen (DE); SIGLE, Irina, 72116 Mössingen (DE); ENDERLE, Markus, D., 72070 Tübingen (DE)
(74) Vertreter: Bohnenberger, Johannes
(86) Internationale Anmeldenummer: PCT/EP2010/069755
(87) Internationale Veröffentlichungsnummer: WO 2011/095253

(56) Entgegenhaltungen:
- WO-A1-93/08897
- CA-C- 1 323 663
- JP-A- H06 178 780
- US-A- 5 707 402
- US-A1- 2006 025 760
- US-A1- 2006 069 387
- US-A1- 2007 135 779
- US-A1- 2008 146 890
- US-A1- 2008 319 441
- US-A1- 2009 187 187
- US-A1- 2014 012 155

## Beschreibung

Die Erfindung betrifft eine elektrochirurgische Anordnung, die ein elektrochirurgisches Instrument und ein mit diesem verbindbares Behandlungsgerät zur Bereitstellung der für einen chirurgischen Eingriff erforderlichen Energie, speziell HF-Energie, umfasst. Nachfolgend ist der Begriff "Behandlungsgerät" in einem allgemeineren Sinne als dem des herkömmlichen HF-Generators zu verstehen; hierauf wird weiter unten genauer eingegangen.

Elektrochirurgische Anordnungen dieser Art sind an sich bekannt und seit langem im klinischen und vereinzelt auch im ambulanten Einsatz. Eine Vielzahl von Patentveröffentlichungen der Anmelderin befasst sich mit deren Verbesserung unter verschiedenen Aspekten. Beim Einsatz derartiger Anordnungen entstehen Emissionen, und zwar speziell Rauchgase, welche aus einer großen Zahl verschiedener organischer Moleküle bestehen. Es ist bekannt, diese Rauchgase durch spezielle Absaugvorrichtungen aus der Atmosphäre über dem Behandlungsort zu entfernen, um Beeinträchtigungen der Arbeit des Chirurgen durch eine Verschlechterung der Sichtverhältnisse zu verhindern.

Eine Reduzierung der für die Rauchgasentwicklung ursächlichen Karbonisation erfolgt bereits durch die APC-Technologie, auch das argonunterstützte Schneiden bei der Elektrochirurgie ist eine erste Maßnahme, um Karbonisation so gering wie möglich zu halten.

Eine Rauchgasanalyse vor explosionskritischen elektrochirurgischen Anwendungen erfolgt dahingehend, dass die Wahrscheinlichkeit der Bildung eines explosionsfähigen Gasgemisches auf ein geringes Maß reduziert wird. Dies wird dadurch erreicht, dass dem zu endoskopierenden Patienten vor der Operation keine Nahrung gegeben und eine umfassende Reinigung des Darmes mittels Spülung vorgenommen wird, bevor dann die Darmspiegelung erfolgt.

Weiterhin kann eine Rauchgasanalyse verwendet werden um die Wahrscheinlichkeit eines Brandes oder eines Feuers im Darm oder Transbronchialsystem zu verringern. Zur Vermeidung von Feuer und Brand im Tracheobronchialsystem bei der Anwendung der APC muss die Sauerstoffkonzentration unter 40% liegen. Eine Rauchgasanalyse eignet sich weiterhin zu einer Reduzierung von Gewebekarbonisation und kanzerogener Bestandteile, welche auf der Gewebeoberfläche gebildet werden und im Rauchgas zu finden sind. Schließlich wird die Sicht des Chirurgen auf das Operationsgebiet verbessert, insbesondere in geschlossenen Lumen.

Zum Stand der Technik wird hingewiesen auf die Druckschriften US 2008/319441 A1, US 5 707 402 A, US 2006/069387 A1, US 2009/187187 A1, US 2014/012155 A1, US 2007/135779 A1, WO 93/08897 A1, US 2008/146890 A1, US 2006/025760 A1, JP H06 178780 und CA 1 323 663 A.

Hiervon ist aus der US 2014/012155 A1 eine elektrochirurgische Anordnung bekannt, die ein elektrochirurgisches Instrument mit einer Nachweiseinrichtung zum Nachweis eines vorbestimmten Bestandteils im Bereich eines Behandlungsortes, die einen Sensor zur Erfassung einer optischen Größe am Behandlungsort aufweist, und ein mit dem Instrument verbindbaren Behandlungsgerät mit einem Stromgenerator zur Bereitstellung von HF-Energie umfasst, wobei im Behandlungsgerät Mittel zur Beeinflussung des Behandlungsvorganges in Abhängigkeit von einem Ausgangssignal der Nachweiseinrichtung vorgesehen sind. Ähnliche Anordnungen werden auch in der US 5 707 402 A und US 2006/025760 A1 offenbart.

Der Erfindung liegt die Aufgabe der Bereitstellung einer verbesserten Anordnung der gattungsgemäßen Art zu Grunde, welche insbesondere eine gezieltere Beeinflussung des Schneid- bzw. Behandlungsvorganges bei gleichzeitiger weitgehender Unterbindung von emissionsbedingten Beeinträchtigungen der Arbeit des Chirurgen ermöglicht.

Diese Aufgabe wird durch eine elektrochirurgische Anordnung mit den Merkmalen des Anspruchs 1 gelöst.

Die Erfindung geht von dem grundlegenden Gedanken aus, dass die bei einem elektrochirurgischen Prozess auftretenden Emissionen eine für die Prozessführung charakteristische Zusammensetzung aufweisen. Weiterhin gehört zur Erfindung der Gedanke, diese verfügbaren Informationen zu nutzen, statt - wie bisher praktiziert - die informationstragenden Emissionen vom Behandlungsort ungenutzt zu entfernen. Dies geschieht durch das Vorsehen einer Nachweiseinrichtung zur Analyse der Emissionen (Rauchgase). Schließlich gehört zur Erfindung der Gedanke, das Analyseergebnis zur Steuerung des chirurgischen Prozesses zu nutzen und im Behandlungsgerät entsprechende Mittel vorzusehen. Das Behandlungsgerät kann grundsätzlich unter Beachtung des Analyseergebnisses manuell gesteuert werden, bevorzugt ist aber eine direkte Steuerung durch ein Ausgangssignal der Nachweiseinrichtung.

Durch die Analyse dieser Rauchgase bzw. Aerosole eröffnen sich folgende Möglichkeiten:
a. Karbonisation ist eine ungewollte Nebenerscheinung bei nahezu allen elektrochirurgischen Anwendungen. Karbonisation führt zu verstärkter Entzündung des Gewebes und zu vermehrten post-operativen Problemen. Deshalb ist eine Reduzierung der Karbonisation bei elektrochirurgischen Anwendungen (HF- u. APC-Anwendungen) erstrebenswert. Eine Realisierung ist durch die Bestimmung von verbrennungsrelevanten chemischen Stoffen des Rauchgases und Rückkopplung des Messsignals zur Steuerung des Behandlungsgerätes möglich. Durch Zuführen von bestimmten gasförmigen oder flüssigen Stoffen (Oxidationsmittel für den entstehenden Kohlenstoff) kann das Ausmaß der Karbonisation weiterhin signifikant reduziert werden.
b. Bei elektrochirurgischen Anwendungen besteht unter bestimmten Bedingungen die Gefahr einer Gasexplosion, einer Verpuffung oder eines Brandes. Durch Analyse der Gasatmosphäre im OP-Situs oder die Analyse des Rauchgases bei elektrochirurgischen Anwendungen kann eine Explosion, eine Verpuffung oder ein Brand wirksam verhindert werden, indem die HF-Energie nur bei Vorliegen eines nicht-explosiven Gasgemisches freigegeben wird. Ein Beispiel hierfür ist die Verhinderung einer Kolonexplosion durch die Analyse und Auswertung der im Kolon vorhandenen brennbaren Gase Methan und Wasserstoff. Eine weitere Anwendungsmöglichkeit bietet die Urologie bezüglich einer APC-Anwendung unter Wasser, bei welcher erhebliche Mengen an Wasserstoff gebildet werden.
c. Die Vaporisation von biologischem Gewebe ist ein wünschenswerter Effekt im Bereich der Tumorentfernung und in anderen Bereichen, in denen biologisches Gewebe restlos entfernt werden soll. Etabliert ist die Vaporisation im Bereich der Laseranwendung. Durch eine gezielte, ortsaufgelöste, möglichst stöchiometrische Verbrennung von biologischem Gewebe soll eine selektive Vaporisation von Gewebe auch in der Elektrochirurgie erreicht werden. Diese Vaporisation soll primär für die Abtragung von Tumorgewebe eingesetzt werden. Hierfür ist die gezielte und ortsaufgelöste Applikation eines Oxidationsmittels (z.B. Sauerstoff) nötig.
d. Rauchgase können aus einer großen Zahl unterschiedlicher organischer Moleküle, Tumormarker, Metaboliten, DNA, Membranmoleküle, Peptide, Proteine und Viren bestehen. Durch Analyse des Rauchgases können Markermoleküle analysiert werden, welche es z.B. erlauben, eine Gewebedifferenzierung vorzunehmen. So kann gesundes von erkranktem (z.B. tumorösem) Gewebe unterschieden oder eine Tiefenwirkung durch Detektion bestimmter Stoffe im Wandaufbau von Schleimhäuten (Magen, Ösophagus, Darm) detektiert werden. Dies hat eine erhöhte Sicherheit bezüglich unerwünschten Tiefenschädigungen und Perforation zur Folge.

Messprinzipien der Sensoren können chemischer, elektrochemischer, spektroskopischer, physikalischer oder physikalisch-chemischer Natur sein. Beispiele hierfür sind Messwerterfassung über Brennstoffzellen, Paramagnetismus, elektrochemische Messzellen, Pellistoren, piezoelektrische Bauelemente, elektrischer Widerstand, Absorption von Strahlung, Feuchtigkeit, Licht, Wärmestrahlung, stoffliche Beschaffenheit einer Umgebung, Abstand, Dehnung, Durchfluss, Farbe, Magnetfeld oder pH-Wert.

Die Sensoren mit zugehöriger Sensorleitung können in das chirurgische Instrument integriert oder extern an diesem befestigt und insbesondere durch eine semipermeable Membran am distalen Ende des chirurgischen Instrumentes vor dem Eindringen von schädlichen Substanzen geschützt sein.

Eine Ausführung der Erfindung sieht vor, dass mindestens ein Teil der Nachweiseinrichtung in einem distalen Bereich des elektrochirurgischen Instruments angeordnet ist. Alternativ hierzu kann vorgesehen sein, dass mindestens ein Teil der Nachweiseinrichtung in einem proximalen Bereich des elektrochirurgischen Instruments oder entfernt vom Instrument angeordnet ist und das Instrument einen ersten Fluidkanal zum Hindurchleiten von Gas zur Nachweiseinrichtung aufweist.

Eine weitere bevorzugte Ausführung der Erfindung sieht vor, dass die Nachweiseinrichtung einen Rauchgasdetektor, insbesondere einen H₂- oder CH₄-Detektor oder Markermolekül-Detektor, aufweist. Die konkrete Ausführung des Detektors kann auf die weiter oben genannten Sensorprinzipien zurückgreifen, wobei insbesondere kompakte und kostengünstige kommerziell verfügbare Detektoren bevorzugt sein werden.

Eine weitere Ausgestaltung der Erfindung sieht vor, dass die Nachweiseinrichtung Mittel zum Abscheiden und eine Analyseeinrichtung zur Analyse von mit dem Gas transportiertem Aerosol oder Feststoffpartikeln aufweist. Hierdurch kann im oben erwähnten Sinne einer Gewebedifferenzierung und Tumorerkennung zusätzliche Information bereitgestellt werden, die nicht unmittelbar und notwendigerweise zur Steuerung des laufenden chirurgischen Prozesses genutzt wird. Teile der Nachweiseinrichtung können dabei auch entfernt vom Behandlungsort und vom im Einsatz befindlichen Instrument, etwa in einem Analyselabor, angeordnet sein und werden vorliegend gleichwohl als Komponente der elektrochirurgischen Anordnung verstanden.

Die Erfindung sieht vor, dass die Nachweiseinrichtung einen Sensor zur Erfassung einer optischen Größe am Behandlungsort, und einen Abstandsfühler zur Erfassung eines Abstandes zwischen dem distalen Ende des elektrochirurgischen Instruments und einem zu behandelnden Gewebe aufweist. Hierbei ergibt sich eine Verbindung zu den o. g. Sensorprinzipien, und zwar über den Nachweis chemischer Verbindungen in der Atmosphäre am Behandlungsort hinausgehend. Beispielsweise kann in einem weiteren Kanal ein Lichtwellenleiter angebracht sein, der es erlaubt, ein optisches Messsignal außerhalb des endoskopischen Instrumentes z.B. mit Hilfe der UV-Vis-Spektroskopie zu analysieren.

Gemäß der Erfindung weisen die Mittel zur Beeinflussung des Behandlungsvorganges eine Fluidquelle zur Bereitstellung eines zur Beeinflussung des Behandlungsvorganges geeigneten Behandlungs-Fluids, speziell eine Edelgasflasche auf, und das elektrochirurgische Instrument weist einen zweiten Fluidkanal zum Hindurchleiten des Behandlungs-Fluids zur Beeinflussung des Behandlungsvorganges zum distalen Ende des Instruments auf.

Insbesondere kann das chirurgische Instrument zusätzlich eine oder mehrere Öffnungen aufweisen, die seitlich und/oder frontal das Absaugen oder das Einbringen von gasförmigen oder flüssigen Stoffen erlauben. Damit kann beispielsweise ein geeignetes flüssiges oder gasförmiges Oxidationsmittel, wie Wasser oder Sauerstoff, zur Reduzierung der Karbonisation (siehe weiter oben) eingebracht werden, was zu einem besseren post-operativen Heilungsprozess führt. Ebenfalls kann der eingebrachte Stoff eine Kühlwirkung herbeiführen, welche sich auf den Gewebeeffekt auswirkt. Diese Öffnungen können verschiedene Ausführungsformen haben, beispielsweise rund, oval oder halbrund sein. Die Öffnungen an dem distalen Sondenende können so gestaltet sein, dass ein Aerosol eines flüssig zugeführten Stoffes erzeugt und auf das biologische Gewebe im Bereich der elektrochirurgischen Anwendung appliziert werden kann.

Des Weiteren kann vorgesehen sein, dass im elektrochirurgischen Instrument oder in Fluidverbindung mit diesem eine Fördereinrichtung zum Fördern von Gas in distal-proximaler Richtung und/oder eine Fördereinrichtung zum Fördern eines Behandlungs-Fluids zur Beeinflussung des Behandlungsvorganges in proximaldistaler Richtung vorgesehen ist. Die letztere Fördereinrichtung kommt insbesondere zur Zuführung eines nicht in einem Druckgefäß bereitgestellten Behandlungs-Fluids zum Einsatz, so etwa bei der Zuführung einer Kochsalzlösung aus einem entsprechenden Tank.

Sowohl bei Einsatz eines unter Druck stehenden Behandlungs-Fluids als auch bei Vorsehen einer Fördereinrichtung für das Behandlungs-Fluid sind bevorzugt Durchflusssteuermittel zur Steuerung der dem Behandlungsort pro Zeiteinheit zugeführten Menge des zur Beeinflussung des Behandlungsvorganges geeigneten Behandlungs-Fluids vorgesehen. Diese Durchflusssteuermittel wirken im Sinne der weiter oben erwähnten Mittel zur Beeinflussung des Behandlungsvorganges, zusammen mit Mitteln zur Steuerung der Zuführung von Behandlungsenergie, speziell HF-Energie. Als derartiges Mittel umfasst die Anordnung eine mit dem Ausgang der Nachweiseinrichtung signalmäßig verbundene Steuereinrichtung des Stromgenerators, insbesondere eine Ein-/Aus-Steuerung und/oder Leistungssteuerung.

Der oben erwähnte Fluidkanal ist in einer weiteren Ausführung der Erfindung innerhalb einer elektrochirurgischen Elektrode des Instruments angeordnet. Beispielsweise kann durch eine solche Zuleitung ein Sauerstoffeintrag bzw. der Eintrag eines Sauerstoffgemisches mit anderen gasförmigen Stoffen mit geeignetem Flow erfolgen, welcher nachhaltig zu einer Vaporisation von biologischem Gewebe führt. Der Sensor kann die Konzentration verbrennungsrelevanter Moleküle detektieren, und durch Regelung der Generatorleistung und/oder des Gasflows wird die Vaporisation des Gewebes maximiert und die Karbonisation minimiert. Damit wäre eine Tumorablation über APC- oder HF-Technik denkbar.

Die in den oben genannten Ausführungen dem Instrument zuzuordnenden Merkmale, insbesondere der erste und/oder zweite Fluidkanal und/oder eine integrierte Nachweiseinrichtung, kennzeichnen zugleich das elektrochirurgische Instrument als relativ selbstständiges Gerät bzw. Produkt, ebenso wie die dem Behandlungsgerät zuzuordnenden Merkmale jenes Gerät als selbstständige Einheit kennzeichnen.

Vorteile und Zweckmäßigkeiten der Erfindung ergeben sich im Übrigen aus der nachfolgenden Beschreibung bevorzugter Ausführungsbeispiele anhand der Figuren. Von diesen zeigen:
Fig. 1 eine schematische Gesamtansicht einer nicht anspruchsgemäßen elektrochirurgischen Anordnung (vgl. hierzu auch Fig. 6),
Fig. 2A - 2D in Detailansichten Ausführungsbeispiele eines elektrochirurgischen Instruments als Komponente einer solchen Anordnung, jeweils in einer Längsschnittdarstellung des distalen Endes und einer Draufsicht von distal,
Fig. 3A und 3B Detailansichten weiterer Ausführungsformen des elektrochirurgischen Instruments, jeweils als Längsschnittdarstellung des distalen Endes in einer Anwendungssituation an einem Behandlungsort,
Fig. 4A - 4D Detailansichten weiterer Ausführungsformen des elektrochirurgischen Instruments, jeweils als Längsschnittdarstellung des distalen Endes in einer Anwendungssituation an einem Behandlungsort,
Fig. 5A und 5B jeweils eine Draufsicht sowie eine Detailansicht (Längsschnitt des distalen Endes) weiterer Ausführungen des elektrochirurgischen Instruments,
Fig. 6 ein Funktions-Blockschaltbild einer Ausführungsform der elektrochirurgischen Anordnung,
Fig. 7A - 7C Impulsdiagramme zur Verdeutlichung von Ausgestaltungen der Betriebsweise eines erfindungsgemäßen Behandlungsgeräts und
Fig. 8A und 8B schematische Darstellungen von beispielhaften Auswertungseinrichtungen einer erfindungsgemäßen elektrochirurgischen Anordnung.

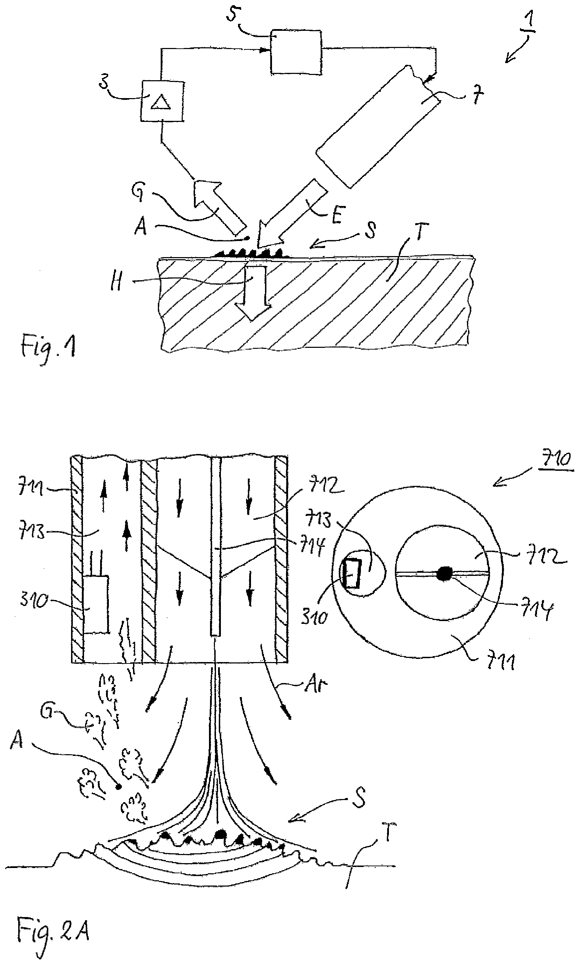

Fig. 1 zeigt schematisch eine elektrochirurgische Anordnung 1 im Einsatz an einem Behandlungsort S an biologischem Gewebe T, wobei durch die Beaufschlagung mit einer Behandlungsenergie E ein Wärmeeintrag H in das Gewebe bewirkt wird und vom Behandlungsort S in die darüber befindliche Atmosphäre A Emissionen, insbesondere Rauchgase G, abgegeben werden. Die Emissionen werden mittels einer Nachweiseinrichtung 3 nachgewiesen, und deren Ausgangssignal wird einem Behandlungsgerät 5 zugeführt, welches seinerseits ausgangsseitig mit einem elektrochirurgischen Instrument 7 verbunden ist, über das in durch das Behandlungsgerät gesteuerter Weise der Behandlungsvorgang ausgeführt und insbesondere die Behandlungsenergie E ausgegeben wird.

Fig. 2A zeigt im Detail das distale Ende eines Instruments 710 im Einsatz an einem Behandlungsort S. In einem Instrumentenkörper 711 sind zwei Lumen 712, 713 ausgebildet, und im kleineren Lumen 713, durch das die Rauchgase G aus der Atmosphäre A über dem Behandlungsort abgesaugt werden, sitzt ein Rauchgasdetektor 310. Über das größere Lumen 712 wird ein Edelgas, z. B. Argon, zur Umspülung einer zentrisch angeordneten HF-Elektrode 714 und zur Beeinflussung des Behandlungsvorganges am Behandlungsort S zugeführt.

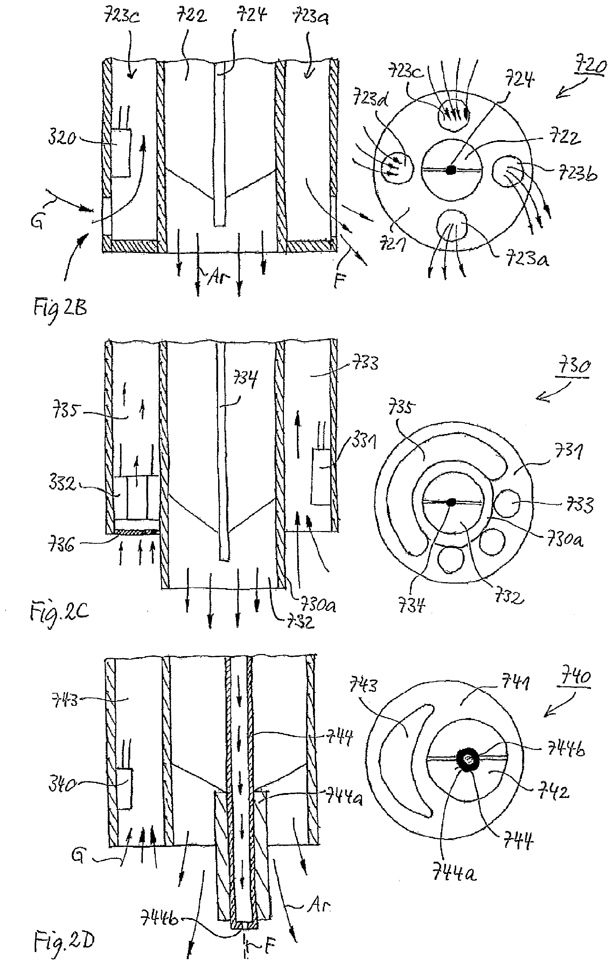

Fig. 2B - 2D zeigen Abwandlungen der Konstruktion des in Fig. 2A gezeigten Instruments, wobei zueinander funktional korrespondierende Teile mit ähnlichen Bezugsziffern wie in Fig. 2A bezeichnet sind und nachfolgend nicht nochmals erläutert werden.

Der Aufbau des Instruments 720 nach Fig. 2B unterscheidet sich von demjenigen des Instruments 710 zunächst durch eine zentrische Platzierung des größeren Lumens 722 zur Zuführung des Edelgases, wobei die HF-Elektrode 724 widerum konzentrisch im Lumen 722 platziert ist. Gleichmäßig um das Lumen 722 verteilt sind vier kleinere Lumen 723a - 723d angeordnet. Hiervon dienen die beiden Lumen 723c und 723d zur Absaugung von Gasen G aus dem Behandlungsbereich und zu deren Detektierung mittels eines Gasdetektors 320, während die beiden verbleibenden äußeren Lumen 723a und 723b weitere Fluide F (Gase oder Flüssigkeiten, neben dem über das zentrale Lumen zugeführten Edelgas) dem Behandlungsort zuführen. Wie aus dem linken Teil der Figur zu erkennen ist, befinden sich die distalen Mündungen der äußeren Lumen nicht in der Stirnseite des Instruments, sondern in dessen Mantelbereich. Hieraus wird auch deutlich, dass der rechte Teil dieser Figur keine Draufsicht auf das distale Ende des Instruments, sondern eine nahe des distalen Endes gewonnene Querschnittsdarstellung ist.

Fig. 2C zeigt als weitere Abwandlung ein Instrument 730, welches sich von den vorab beschriebenen Instrumenten 710 und 720 zunächst durch ein abgestuftes distales Ende mit vorstehendem Mittenbereich 730a auszeichnet. Des Weiteren sind hier mehrere gleiche kleinere Lumen 733 mit kreisförmigem Querschnitt sowie ein größeres, den Mittenbereich 730a des Instruments partiell C-förmig umgebendes Lumen 735 vorgesehen, in denen verschiedene Detektoren 331 und 332 zum Nachweis verschiedener Bestandteile der Gase G und/oder von weiteren Parametern der Atmosphäre über dem Behandlungsort sitzen. Das C-förmige Lumen 735 ist dabei nach distal durch eine semipermeable Membrane 736 zum Schutz des dort angeordneten Detektors 332 vor aufsteigendem Dampf bzw. Feuchtigkeit am Behandlungsort verschossen.

Fig. 2D zeigt als weitere Abwandlung ein Instrument 740, welches wie die vorgenannten Ausführungen ein großes Lumen 742 zur Zuführung der HF-Energie sowie von Behandlungs-Fluiden aufweist. Neben diesem ist im Instrumentenkörper 741 lediglich ein einzelnes weiteres Lumen 743 ausgebildet, welches hier Sichelform hat und in dem - ähnlich wie bei der ersten Ausführungsform nach Fig. 2A - ein einzelner Detektor 340 zur Analyse der Gase G platziert ist.

Eine wesentliche Modifikation gegenüber dem Instrument 710 besteht darin, dass die HF-Elektrode 744 hier als Metallrohr ausgebildet ist, durch das ein Behandlungs-Fluid, insbesondere gasförmiger Sauerstoff oder eine NaCI-Lösung, zum Behandlungsort geleitet werden kann. Zudem ist die Elektrode 744 hier distal über das Instrumentenende vorstehend ausgeführt und hat in ihrem Endbereich eine thermisch belastbare Ummantelung 744a aus einem elektrischen Isolator, um eine (zusätzliche) Karbonisation durch starke Lichtbögen bei einer APC-Anwendung zu vermeiden. Als Materialien für die Beschichtung 744a kommen Keramiken oder auch hochtemperaturfeste Kunststoffe, etwa auf PTFE-Basis, in Betracht. Eine Düse 744c in der Stirnseite des Elektroden-Rohres 744 sorgt für ein Zersprühen einer zugeführten Flüssigkeit über dem Behandlungsort.

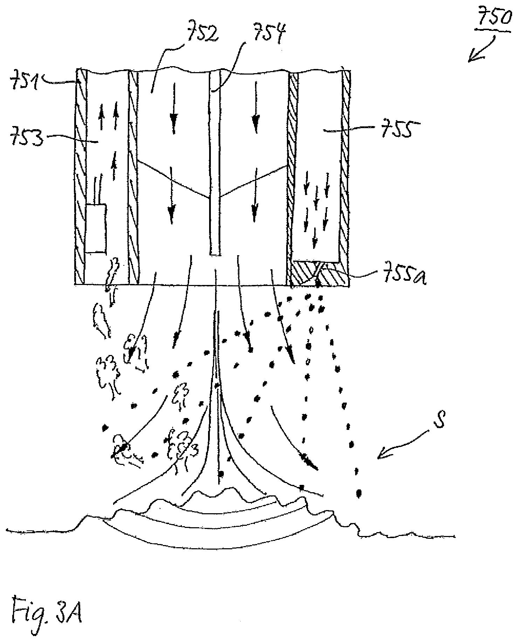

Fig. 3a zeigt als weitere Ausführungsform ein Instrument 750 im Einsatz an einem Behandlungsort S, welches wiederum mehrere Lumen 725, 753 und 755 aufweist. Hinsichtlich des Aufbaus und der Funktion der Lumen 752, 753 und der im Lumen 752 angeordneten HF-Elektrode 754 entspricht das Instrument 750 dem in Fig. 2A gezeigten und weiter oben beschriebenen Instrument 710. Hinzugefügt ist mit dem Lumen 755 und dessen schräg und sich nach außen erweiternd verlaufender distaler Öffnung 755a eine Einrichtung zum Zuführen und gezielten und gegebenenfalls sprühenden Austrag eines Behandlungs-Fluids, wie etwa einer physiologisch wirksamen wässrigen Lösung und/oder eines Oxidationsmittels. Neigung und Gestalt der Öffnung 755a sind derart bemessen, dass das Behandlungs-Fluid unter einem geeigneten Druck und fokussiert über dem Behandlungsort S ausgetragen wird.

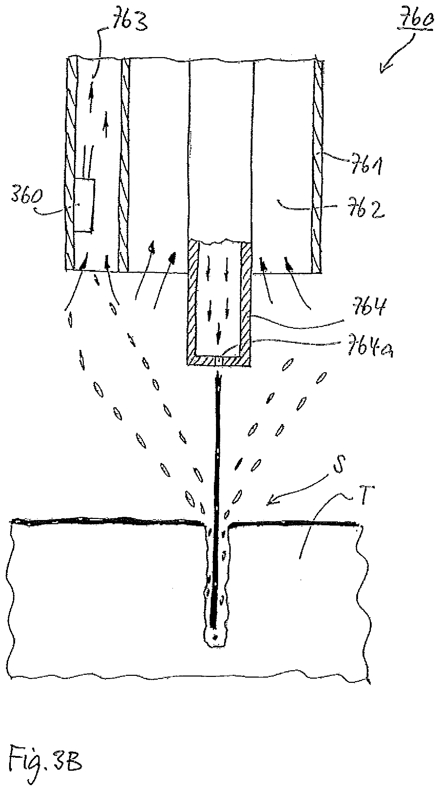

Fig. 3B zeigt, als Modifikation des in Fig. 2D gezeigten und weiter oben beschriebenen Instruments 740, in schematischer Darstellung einen mehrlumigen Wasserstrahl-Applikator 760 mit innenliegender aktiver HF-Elektrode 764, die als Metallrohr ausgebildet ist, durch welches Flüssigkeit mit hoher Fliessgeschwindigkeit durch eine Düsenöffnung 764a ausströmt, um den gewünschten Effekt in dem Gewebe zu erzielen. Eingebrachte Flüssigkeit und Blut wird bei Aufsetzen der Sonde auf das Gewebe in den Absaugkanal 762 abgesaugt. Über einen weiteren Kanal 763 werden Substanzen, z.B. Aerosole, parallel zur Anwendung durch Unterdruck durch das Instrument vorbei an einem Sensor 360, der am distalen Ende des Instrumentes befestigt ist, abgesaugt und analysiert.

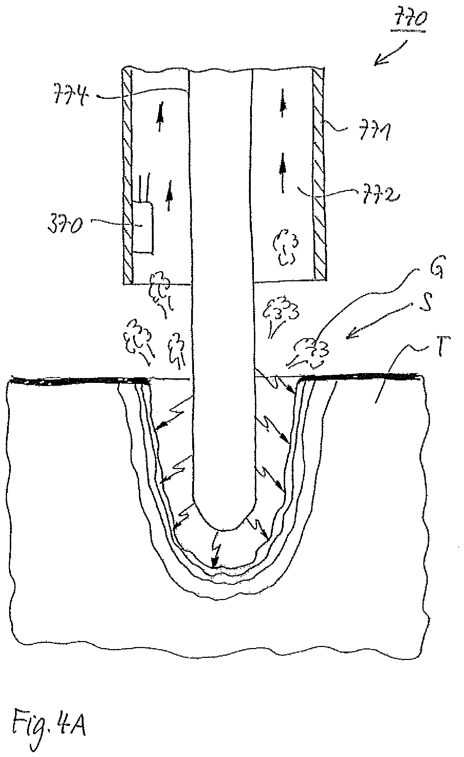

Fig. 4A zeigt eine schematische Darstellung eines monopolaren HF-Instrumentes 770 zum Schneiden von biologischem Gewebe, in dem die aktive Elektrode 774 mit einem Rohr 761 umhüllt ist, über das das entstehende Rauchgas G abgesaugt werden kann. In dem Rohr, das z.B. zentral oder parallel zur Achse der aktiven Elektrode angebracht sein kann, befindet sich am distalen Ende ein Sensor 370 für die Rauchgasanalyse.

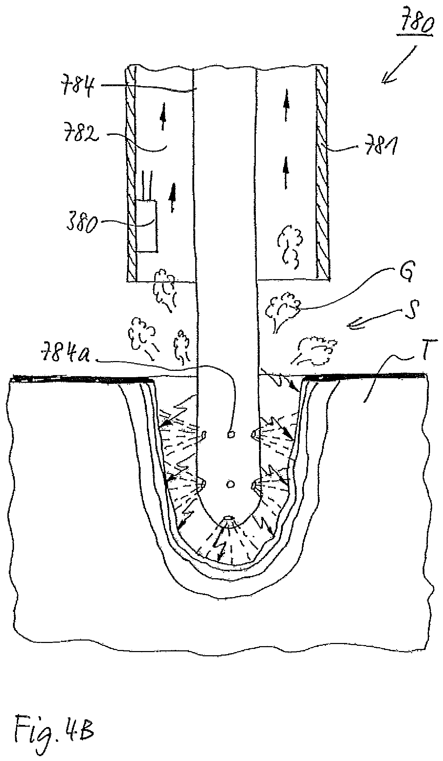

Fig. 4B zeigt eine schematische Darstellung eines weiteren monopolaren HF-Instrumentes 780 zum Schneiden von biologischem Gewebe, in dem die aktive Elektrode 784 an der Spitze kleine Öffnungen 784a besitzt, über die flüssige oder gasförmige Stoffe austreten können, und das so ausgebildet ist, dass über ein Absaugrohr 782, die Rauchgase G oder Aerosole abgesaugt und über einen Sensor 380 analysiert werden können.

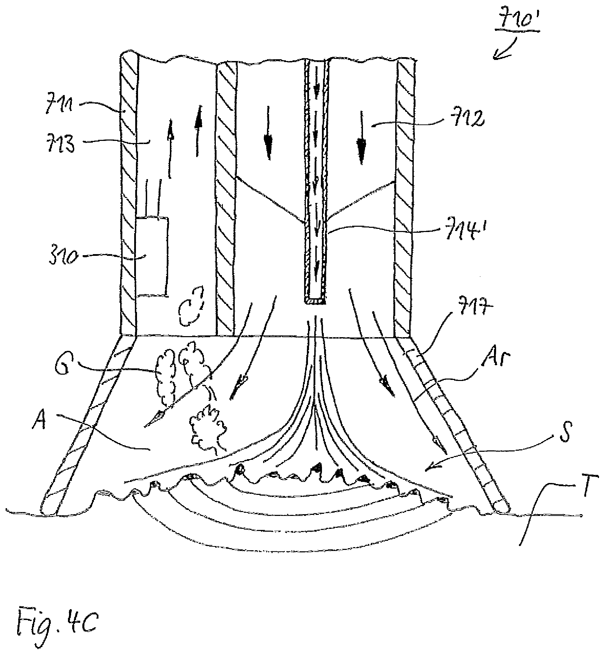

Fig. 4C zeigt den Einsatz eines gegenüber der Ausführung nach Fig. 2A etwas modifizierten Instruments 710'. Dieses unterscheidet sich vom Instrument 710 zum einen durch eine hohle Ausführung der Elektrode 714', in deren Innerem ein Behandlungs-Fluid dem Behandlungsort S zugeführt wird, und des Weiteren durch die distale Anbringung eines sich kegelförmig erweiternden Aufsatzes 717, der die Atmosphäre A über dem Behandlungsort S weitgehend von der übrigen Atmosphäre trennt. Dieser Aufsatz 717 vermeidet somit mit hoher Zuverlässigkeit den Austritt von Rauchgasen in den OP-Raum und ermöglicht zudem eine effizientere Ausnutzung der zugeführten Behandlungs-Fluide.

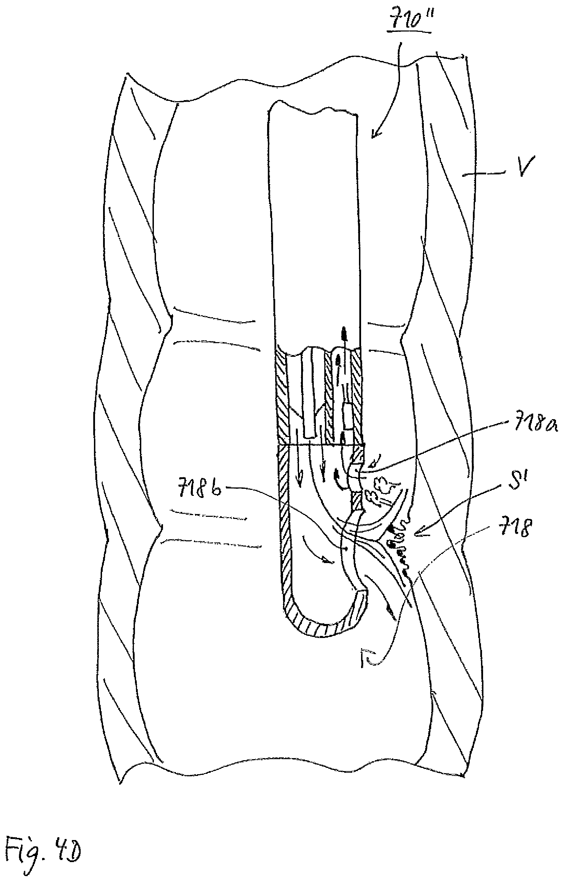

Fig. 4D zeigt skizzenartig den Einsatz eines Instruments 710" an einem Behandlungsort S' in einem Hohlorgan V (z.B. Darm) eines Patienten. Die besondere konstruktive Ausgestaltung dieses Instruments 710" besteht in einer distal aufgesetzten Kappe 718 mit zwei seitlichen Öffnungen 718a, 718b, über die Rauchgase in das Innere des Instruments abgesaugt bzw. Behandlungs-Fluide aus dem Instrument an den Behandlungsort S' zugeführt werden. Im Übrigen entspricht der Aufbau demjenigen des vorstehend beschriebenen Instruments 710' bzw. des weiter oben erläuterten Instruments 710 und wird insoweit nicht nochmals beschrieben.

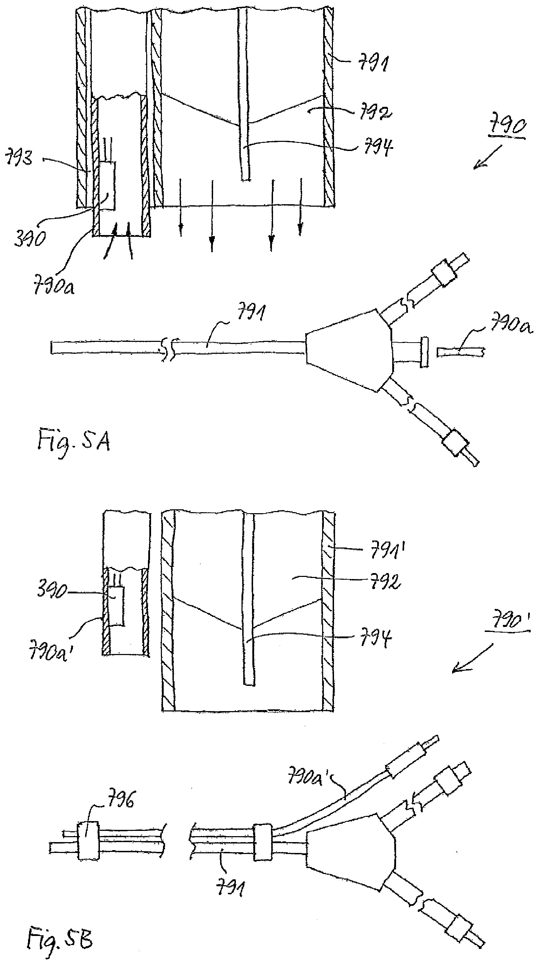

Fig. 5 zeigt ein APC-Instrument 790, dessen Instrumentenkörper wiederum ähnlich wie bei den weiteren oben beschriebenen Instrumenten aufgebaut ist, so dass die Bezugsziffern für die einzelnen Teile bzw. Bereiche an Fig. 2A - 3B angelehnt sind. Die größte Ähnlichkeit weist das Instrument 790 zum Instrument 710 nach Fig. 2A auf; ein wesentlicher Unterschied gegenüber diesem besteht im Vorsehen einer separaten Messsonde 790a, die im Lumen 793 proximal-distal verschieblich angeordnet ist und den darin montierten Detektor 390 somit besonders nahe an einen Behandlungsort führen kann.

Fig. 5B zeigt als Abwandlung dieser letztgenannten Ausführung ein weiteres APC-Instrument 790', bei dem eine verschiebliche Messsonde 790a' mit dem Detektor 390 außen an einem modifizierten (einlumigen) Instrumentenkörper 791' verschieblich angebracht ist. Zur Halterung der Sonde 790a' am Grundkörper 791' des Instruments dienen Befestigungsschellen 796, in denen die Sonde gleiten kann.

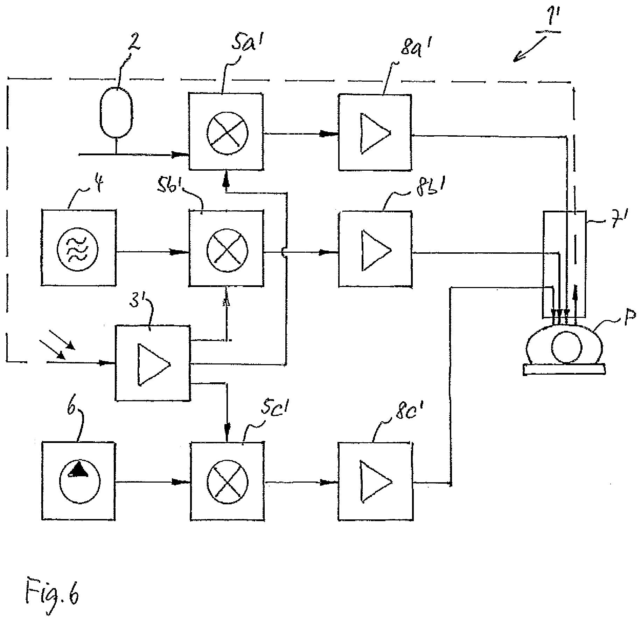

Fig. 6 zeigt schematisch in Form eines Funktions-Blockschaltbildes eine elektrochirurgische Anordnung 1' mit einem chirurgischen Instrument 7', deren Grundaufbau und Einsatz der in Fig. 1 gezeigten Anordnung entspricht und die auf einen Patienten P angewandt wird. Ein Emissionssensor 3' mit zugeordnetem Messverstärker führt einen vorbestimmten Nachweisvorgang am Behandlungsort aus, etwa eine Rauchgasanalyse, eine CO-Erfassung, eine Temperatur-Erfassung oder auch eine Erfassung des Abstandes zwischen Instrumentenende und Gewebe etc., und sein Ausgangssignal wird, mit dem Ziel einer geeigneten Beeinflussung des Behandlungsvorganges, verschiedenen Steuereinrichtungen zugeführt. Es handelt sich hierbei um eine Durchflusssteuerung 5a' für den Gasstrom eines aus einer Gasflasche 2 zugeführten Behandlungs-Gases, um eine HF-Steuereinrichtung 5b' zur Steuerung der von einem HF-Generator 4 bereitgestellten Behandlungsenergie und um eine Spülflüssigkeits-Steuerung 5c' zur Durchflusssteuerung einer aus einer Quelle 6 bereitgestellten Spülflüssigkeit. In der Figur ist schematisch zudem dargestellt, dass in allen "Kanälen" zur Beeinflussung des Behandlungsvorganges zusätzliche Verstärker- bzw. Steuereinrichtungen 8a', 8b' und 8c' vorgesehen sein können.

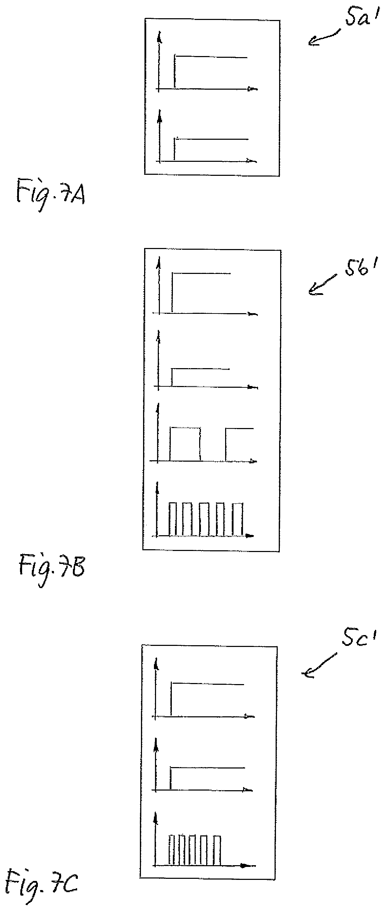

Fig. 7A - 7C zeigen beispielhaft in Art von Impulsdiagrammen Möglichkeiten der Modulation des Behandlungsgasstromes durch die erste Steuereinrichtung 5a' (Fig. 7A), der HF-Generatorleistung durch die zweite Steuereinrichtung 5b' (Fig.7B) und der Bereitstellung von Spülflüssigkeit durch die dritte Steuereinrichtung 5c' (Fig. 7C).

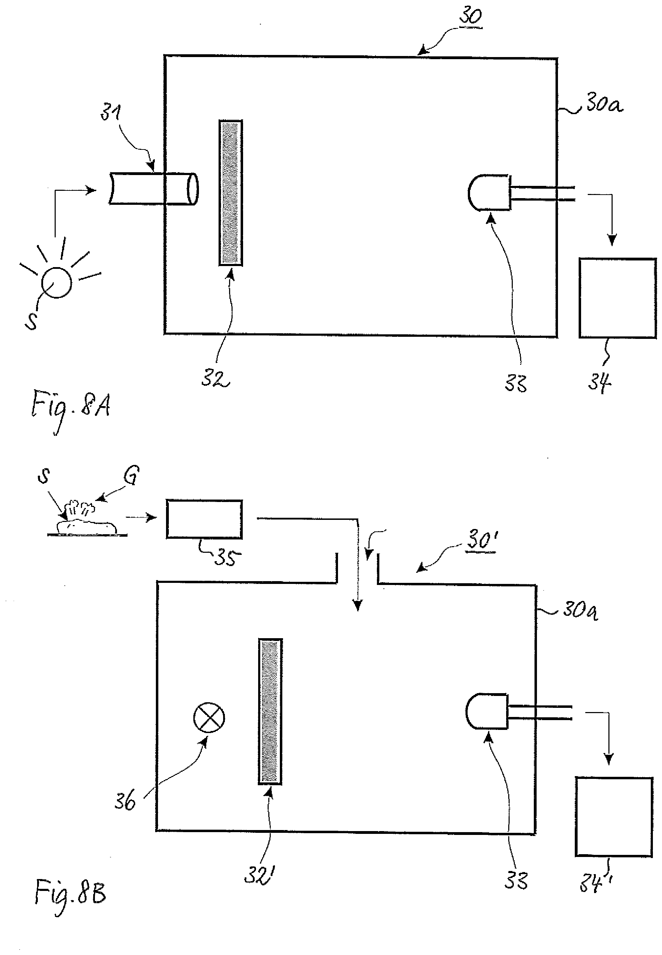

Fig. 8A zeigt eine Prinzipskizze eines Sensorsystems 30, bei welchem die Lichtemission am Behandlungsort S (im sichtbaren oder ultravioletten Wellenlängenbereich) in einen Lichtwellenleiter 31 eingekoppelt wird. Diese polychromatische Lichtemission wird mit Hilfe eines optischen Filters 32 derart gefiltert, dass nur noch eine Wellenlänge oder ein schmales Band von Wellenlängen, welche für den zu beobachtenden Prozess (z.B. den Prozess der Verkohlung der Gewebeoberfläche) charakteristisch ist, durchgelassen wird. Diese Wellenlänge oder dieses Wellenlängenband wird von einer geeigneten Fotodiode 33 detektiert und in ein Spannungssignal umgewandelt, wobei das Spannungssignal proportional zur Intensität der gefilterten Wellenlänge oder des Wellenlängenbandes ist. Dieses Spannungssignal wird von einer Auswerteeinheit 34 ausgewertet und zur Steuerung/Regelung des HF-Generators und der Additivzufuhr verwendet.

Fig. 8B zeigt eine Prinzipskizze eines weiteren Sensorsystems 30', bei welchem das entstehende Rauchgas einer elektrochirurgischen Anwendung mittels einer Pumpe 35 durch eine (nicht gezeigte) Schlauchleitung in das Sensorgehäuse 30a eingebracht wird. Im Sensorgehäuse befinden sich eine polychromatische oder eine monochromatische Lichtquelle 36, ein optisches Filter 32' und eine Fotodiode 33. Das optische Filter filtert eine optische Wellenlänge oder ein Wellenlängenband aus dem polychromatischen Spektrum der Lichtquelle heraus. (Bei Verwendung einer monochromatischen Lichtquelle kann es entfallen). Die das Filter verlassende Strahlung trifft auf Moleküle des Rauchgases G und wird von diesen ganz oder teilweise absorbiert. Die verbleibende Strahlungsintensität trifft auf die Fotodiode, welche in Abhängigkeit der einfallenden Strahlungsintensität ein Spannungssignal erzeugt. Dieses Spannungssignal wird von einer Auswerteeinheit 34' ausgewertet und zur Steuerung/Regelung des HF-Generators und der Additivzufuhr verwendet.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen der Ansprüche liegen.

## Patentansprüche

1. Elektrochirurgische Anordnung, mit
einem elektrochirurgischen Plasmakoagulationsinstrument,
einer Nachweiseinrichtung zum Nachweis eines vorbestimmten Bestandteils der Atmosphäre im Bereich eines Behandlungsortes, die einen Sensor zur Erfassung einer optischen Größe am Behandlungsort und einen Abstandsfühler zur Erfassung eines Abstandes zwischen dem distalen Ende des elektrochirurgischen Instruments und einem zu behandelnden Gewebe aufweist, und
einem mit dem Instrument verbindbaren Behandlungsgerät mit einem Stromgenerator zur Bereitstellung von HF-Energie, wobei im Behandlungsgerät Mittel zur Beeinflussung des Behandlungsvorganges in Abhängigkeit von den Ausgangssignalen des Sensors und Abstandsfühlers der Nachweiseinrichtung vorgesehen sind,
wobei die Mittel zur Beeinflussung des Behandlungsvorganges eine mit dem Ausgang der Nachweiseinrichtung signalmäßig verbundene Steuereinrichtung des Stromgenerators sowie eine Fluidquelle zur Bereitstellung eines zur Beeinflussung des Behandlungsvorganges geeigneten Behandlungs-Fluids aufweisen, wobei die Fluidquelle eine Edelgasflasche ist und wobei die Steuereinrichtung zur Steuerung der von dem Stromgenerator bereitgestellten Behandlungsenergie ausgebildet ist und
wobei das Instrument einen ersten Fluidkanal zum Hindurchleiten des Behandlungs-Fluids zum distalen Ende des Instruments aufweist.

2. Elektrochirurgische Anordnung nach Anspruch 1, wobei mindestens ein Teil der Nachweiseinrichtung in einem distalen Bereich des Instruments angeordnet ist.

3. Elektrochirurgische Anordnung nach Anspruch 1, wobei mindestens ein Teil der Nachweiseinrichtung in einem proximalen Bereich des Instruments oder entfernt vom Instrument angeordnet ist und das Instrument einen zweiten Fluidkanal zum Hindurchleiten von Gas zur Nachweiseinrichtung aufweist.

4. Elektrochirurgische Anordnung nach einem der vorangehenden Ansprüche, wobei die Nachweiseinrichtung Mittel zum Abscheiden und eine Analyseeinrichtung zur Analyse von mit dem Gas transportiertem Aerosol oder Feststoffpartikeln aufweist.

5. Elektrochirurgische Anordnung nach einem der vorangehenden Ansprüche, wobei im Instrument oder in Fluidverbindung mit diesem eine erste Fördereinrichtung zum Fördern eines Behandlungs-Fluids in proximaldistaler Richtung und/oder eine zweite Fördereinrichtung zum Fördern von Gas in distal-proximaler Richtung vorgesehen ist.

6. Elektrochirurgische Anordnung nach Anspruch 5, mit Durchflusssteuermitteln zur Steuerung der dem Behandlungsort pro Zeiteinheit zugeführten Menge des Behandlungs-Fluids.

7. Elektrochirurgische Anordnung nach einem der vorangehenden Ansprüche, wobei das Instrument am oder nahe dem distalen Ende eine mit dem ersten Fluidkanal verbundene Vielzahl von Öffnungen zum räumlich verteilten Auslass des Behandlungsfluids aufweist.

8. Elektrochirurgische Anordnung nach einem der vorangehenden Ansprüche, wobei der erste Fluidkanal innerhalb einer elektrochirurgischen Elektrode des Instruments angeordnet ist.

9. Elektrochirurgische Anordnung nach einem der vorangehenden Ansprüche, wobei die Steuereinrichtung des Stromgenerators zur Ein-/Aus-Steuerung und/oder Leistungssteuerung der HF-Energie ausgebildet ist.

## Claims

1. Electrosurgical assembly with an electrosurgical plasma coagulation instrument, a detection device for detecting a predetermined constituent of the atmosphere in the region of a treatment site, which detection device has a sensor for sensing an optical variable at the treatment site and a distance sensor for sensing a distance between the distal end of the electrosurgical instrument and a tissue to be treated, and a treatment unit, which can be connected to the instrument and has a power generator for providing RF energy, wherein means for influencing the treatment process in dependence on the output signals of the sensor and distance sensor of the detection device are provided in the treatment unit, wherein the means for influencing the treatment process have a control device of the power generator that is connected in signaling terms to the output of the detection device, and also have a fluid source for providing a treatment fluid suitable for influencing the treatment process, wherein the fluid source is a noble-gas cylinder and wherein the control device is configured to control the treatment energy provided by the power generator and wherein the instrument has a first fluid channel for passing the treatment fluid through to the distal end of the instrument.

2. Electrosurgical assembly according to Claim 1, wherein at least part of the detection device is arranged in a distal region of the instrument.

3. Electrosurgical assembly according to Claim 1, wherein at least part of the detection device is arranged in a proximal region of the instrument or away from the instrument and the instrument has a second fluid channel for passing gas through to the detection device.

4. Electrosurgical assembly according to one of the preceding claims, wherein the detection device has means for depositing and an analysis device for analyzing aerosol or solid particles transported with the gas.

5. Electrosurgical assembly according to one of the preceding claims, wherein a first conveying device for conveying a treatment fluid in the proximal-distal direction and/or a second conveying device for conveying gas in the distal-proximal direction is/are provided in the instrument or in fluid connection with it.

6. Electrosurgical assembly according to Claim 5, with flow control means for controlling the amount of treatment fluid that is supplied to the treatment site per unit of time.

7. Electrosurgical assembly according to one of the preceding claims, wherein the instrument has at or near the distal end a large number of openings connected to the first fluid channel for the spatially distributed outletting of the treatment fluid.

8. Electrosurgical assembly according to one of the preceding claims, wherein the first fluid channel is arranged within an electrosurgical electrode of the instrument.

9. Electrosurgical assembly according to one of the preceding claims, wherein the control device of the power generator is configured for on/off control and/or power output control of the RF energy.

## Revendications

1. Système électrochirurgical, comportant
un instrument électrochirurgical de coagulation par plasma,
un dispositif de détection destiné à détecter un constituant prédéterminé de l'atmosphère dans la région d'un site de traitement, qui comporte un capteur destiné à détecter une grandeur optique sur le site de traitement et un détecteur de distance destiné à détecter une distance entre l'extrémité distale de l'instrument électrochirurgical et un tissu à traiter, et
un appareil de traitement pouvant être relié à l'instrument et comportant un générateur de courant destiné à fournir de l'énergie HF, dans lequel il est prévu dans l'appareil de traitement des moyens permettant d'influencer le processus de traitement en fonction des signaux de sortie du capteur et du détecteur de distance du dispositif de détection,
dans lequel les moyens destinés à influencer le processus de traitement comprennent un dispositif de commande du générateur de courant, relié pour l'échange de signaux à la sortie du dispositif de détection, ainsi qu'une source de fluide destinée à fournir un fluide de traitement approprié pour influencer le processus de traitement, dans lequel la source de fluide est une bouteille de gaz noble et dans lequel le dispositif de commande est conçu pour commander l'énergie de traitement fournie par le générateur de courant et
dans lequel l'instrument comporte un premier canal de fluide destiné à acheminer le fluide de traitement vers l'extrémité distale de l'instrument.

2. Système électrochirurgical selon la revendication 1, dans lequel au moins une partie du dispositif de détection est disposée dans une région distale de l'instrument.

3. Système électrochirurgical selon la revendication 1, dans lequel au moins une partie du dispositif de détection est située dans une région proximale de l'instrument ou éloignée de l'instrument et l'instrument comporte un second canal de fluide destiné à acheminer un gaz vers le dispositif de détection.

4. Système électrochirurgical selon l'une des revendications précédentes, dans lequel le dispositif de détection comprend des moyens de séparation et un dispositif d'analyse permettant l'analyse d'aérosols ou de particules solides transportés avec le gaz.

5. Système électrochirurgical selon l'une des revendications précédentes, dans lequel il est prévu dans l'instrument ou en communication fluidique avec celui-ci un premier dispositif de transport destiné à transporter un fluide de traitement dans la direction proximale-distale et/ou un second dispositif de transport destiné à transporter un gaz dans la direction distale-proximale.

6. Système électrochirurgical selon la revendication 5, comportant des moyens de régulation du débit destinés à commander la quantité de fluide de traitement acheminée par unité de temps vers le site de traitement.

7. Système électrochirurgical selon l'une des revendications précédentes, dans lequel l'instrument comporte à l'extrémité distale ou à proximité de celle-ci une pluralité d'ouvertures reliées au premier canal de fluide pour la sortie spatialement répartie du fluide de traitement.

8. Système électrochirurgical selon l'une des revendications précédentes, dans lequel le premier canal de fluide est disposé dans une électrode électrochirurgicale de l'instrument.

9. Système électrochirurgical selon l'une des revendications précédentes, dans lequel le dispositif de commande du générateur de courant est conçu pour la commande marche/arrêt et/ou la régulation de puissance de l'énergie HF.
